# EUROPEAN PATENT SPECIFICATION

(11) **EP 0 308 220 B1**
(45) Date of publication and mention of the grant of the patent: **08.06.1994**
(21) Application number: 88308532.6
(22) Date of filing: 15.09.1988
(51) Int. Cl.: A61K 39/275, A61K 39/17, A61K 39/295

(54) **Recombinant avipoxvirus**
Rekombinantes Avipoxvirus
Avipoxvirus recombinant

(30) Priority: 16.09.1987 JP 231653/87
(43) Date of publication of application: 22.03.1989
(73) Proprietor: NIPPON ZEON CO., LTD., Tokyo 109 (JP); SHIONOGI & CO., LTD., Osaka 541 (JP)
(72) Inventor: Yanagida, Noboru, Nakahara-ku Kawasaki-shi (JP); Saeki, Sakiko, Ota-ku Tokyo (JP); Ogawa, Ryohei, Isogo-ku Yokohama (JP); Kamogawa, Kouichi, Kanazawa-ku Yokohama (JP); Hayashi, Yoshiyuki, Kusatsu-shi (JP); Sawaguchi, Kazunari, Koga-gun Shiga.ken (JP)
(74) Representative: Paget, Hugh Charles Edward

(56) References cited:
- EP-A- 0 227 414
- EP-A- 0 284 416
- WO-A-86/00528
- WO-A-86/05806
- WO-A-88/02022
- CHEMICAL ABSTRACTS, vol. 108, no. 15, 11th April 1988, page 151, abstract no.125379k, Columbus, Ohio, US; C.D. WEMERS et al.: "The hemagglutinin-neuraminidase (HN) gene of Newcastle disease virus strain Italien (ndvItalien): comparison with HNs of other strains and expression by a vacciniarecombinant"
- CHEMICAL ABSTRACTS, vol. 107, no. 19, 9th November 1987, page 155, abstract no.169718d, Columbus, Ohio, US; E.D. JORGENSEN: "Cloning, nucleotide sequence,and expression of the Newcastle disease virus hemagglutinin-neuraminidase glycoprotein gene"
- J. GEN. VIROL., vol. 67, 1986, pages 1591-1600, GB; D.B. BOYLE et al.:"Identification and cloning of the fowlpox virus thymidine kinase gene using vaccinia virus"
- VACCINES 1986, NEW APPROACHES IN IMMUNOLOGY, 1986, pages 289-292, Cold Spring Harbor Laboratory; F. BROWN et al.: "New vaccinia virus expression vector"

## Description

The present invention relates to a recombinant Avipoxvirus.

In recent years, a method of constructing recombinant vaccinia virus having inserted Newcastle disease virus-derived cDNA into vaccinia virus has been devised and there has been come to propose a method utilizing recombinant vaccinia virus obtained using, as exogenous DNA, for example, DNA coding for infectious diseases as live vaccine (for example, Published Unexamined Japanese Patent Application KOKAI No. 129971/83, Public Disclosure Nos. 500518/85, 501957/86, etc.). According to this method, it is possible to insert a variety of exogenous DNAs depending upon purpose and, the method is expected to be promising as a new process for producing live vaccine.

In vaccinia virus, however, its host range is limited. For this reason, it is almost impossible to apply techniques of recombinant vaccinia virus to production of, for example, live vaccine against chick disease such as Newcastle disease and for the purpose of producing avian live vaccine, a suggested promising method is to insert exogenous DNA into Fowlpoxvirus in lieu of vaccinia virus (Avian Diseases, vol. 30, No. 1, 24-27). However, comparing vaccinia virus and Fowlpoxvirus, they belong to different genera; the former belonging to the genus Poxvirus and the latter to the genus Avipoxvirus. Further, there is a difference in a length of genome by about 1.5 times because the former has a genome length of about 180 Kb and the latter has a genome length of 260 to 270 Kb. Furthermore, with vaccinia virus, its genome DNA structure has been clarified to a remarkable extent, but only restriction enzyme cleavage pattern of genome DNA is known with Fowlpoxvirus (J. Gen. Virol., 38, 135-147 (1977)); turning to function of genome DNA, the presence of thymidine kinase gene is merely proved [J. Gen. Virol., 67, 1591-1600 (1986)].

Accordingly, it is expected that application of the aforesaid method of constructing recombinant vaccinia virus to Avipoxvirus would be accompanied by many difficulties. For this reason, there has recently been found cDNA coding for hemagglutinin neuraminidase (HN) and/or F polypeptide of Newcastle disease virus [for example, J. Gen. Virol., 67, 1917-1927 (1986), Published Unexamined Japanese Patent Application KOKAI No. 163693/87]. However, any recombinant Avipoxvirus having inserted such cDNA thereon has not yet been obtained.

Therefore, the present inventors have made extensive investigations under the state of the art, aiming at construction of a recombinant Avipoxvirus capable of proliferation in which cDNA coding for an antigen derived from Newcastle disease virus (hereafter sometimes referred to as NDV) has been inserted into genome DNA. As a result, the present inventors have found that the recombinant Avipoxvirus capable of proliferation can be obtained by selecting a hemagglutinin neuraminidase (hereafter sometimes referred to as HN)-coding region from NDV cDNA and inserting the region into a genome region non-essential to proliferation of Avipoxvirus and have come to accomplish the present invention.

Thus, according to the present invention, there is provided a vaccine for fowl comprises a recombinant Avipoxvirus in which NDV-derived antigen-encoding cDNA has been inserted into a genome region non-essential to proliferation of Avipoxvirus, preferably together with DNA having a promoter function, in the form capable of expression.

### Brief Description of the Drawings

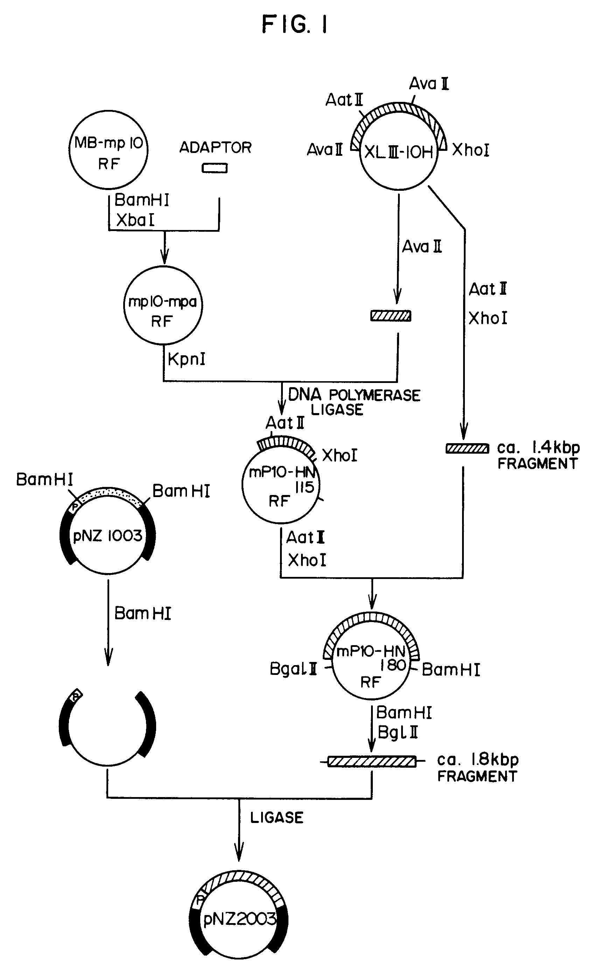

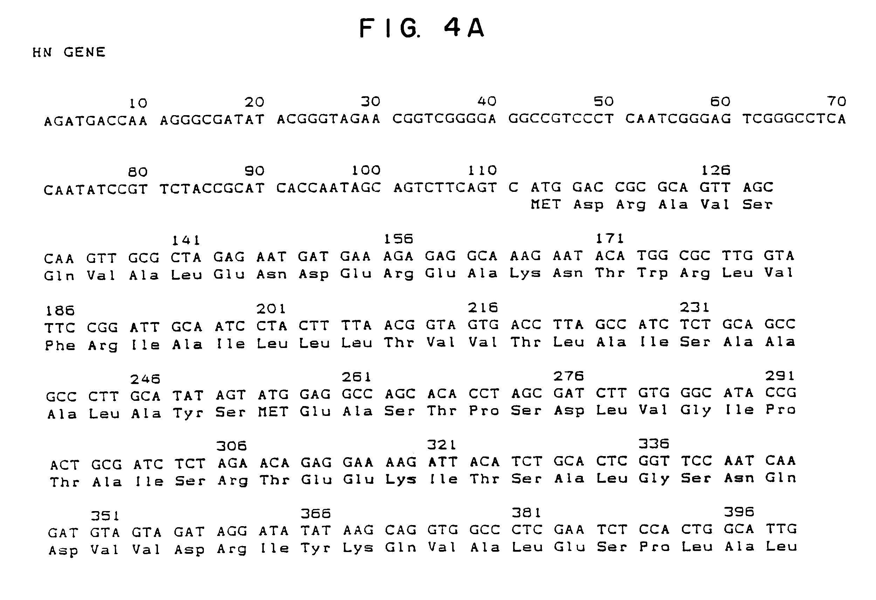

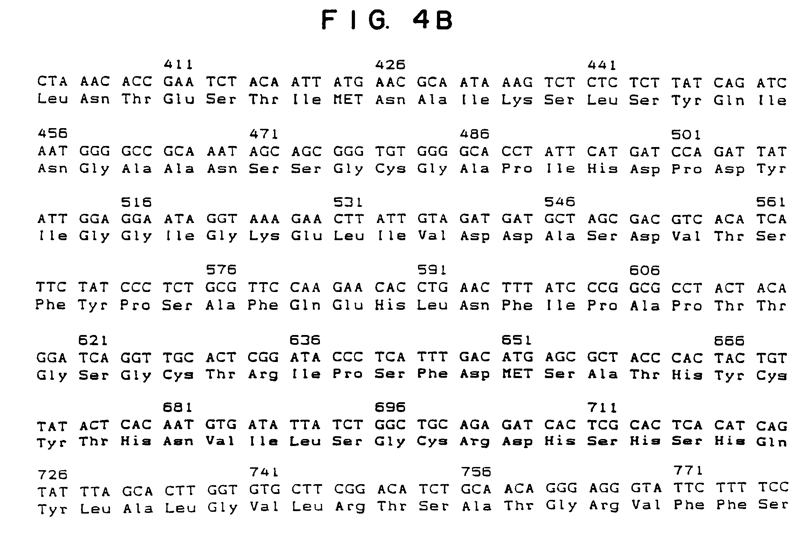

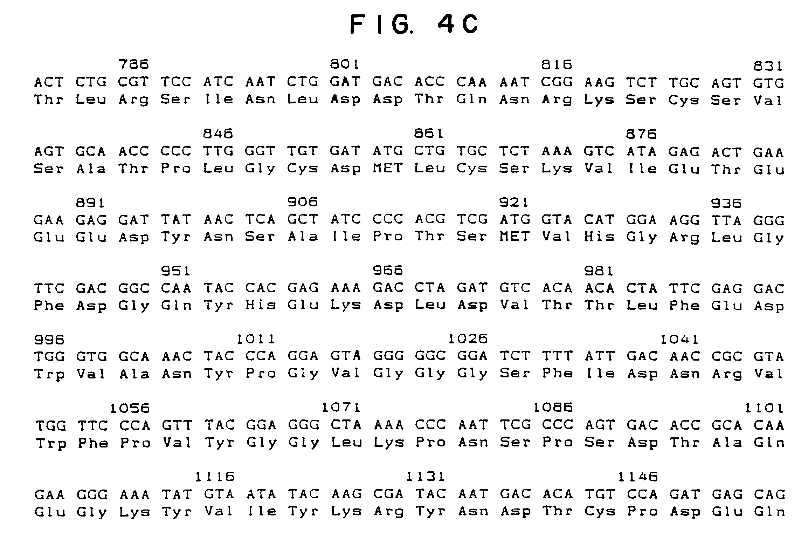

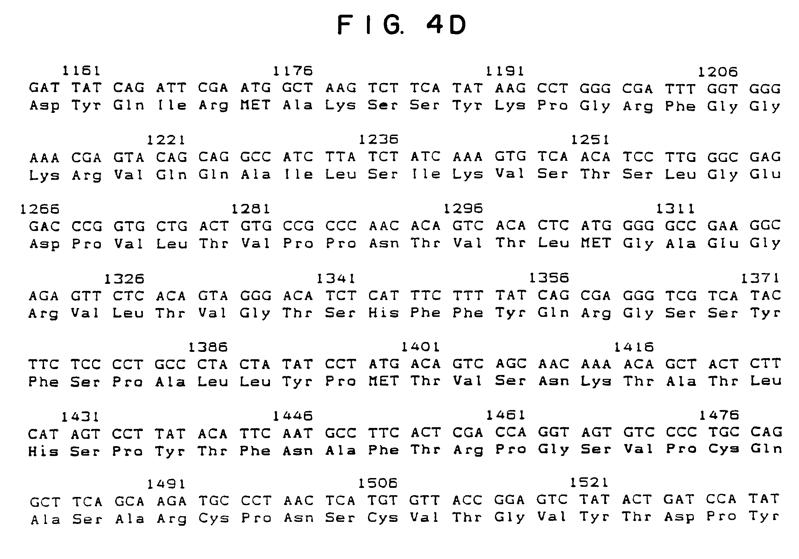

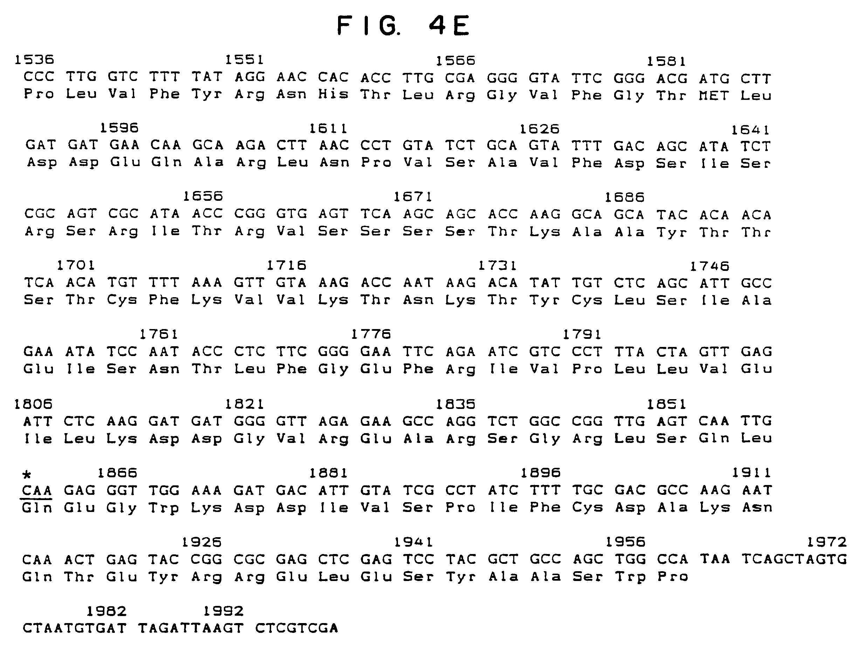

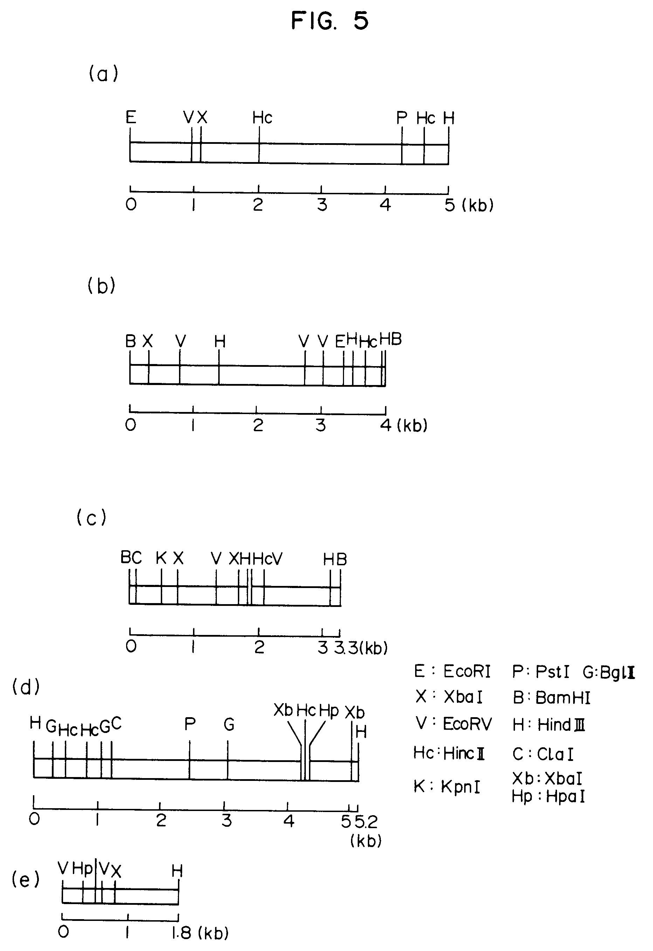

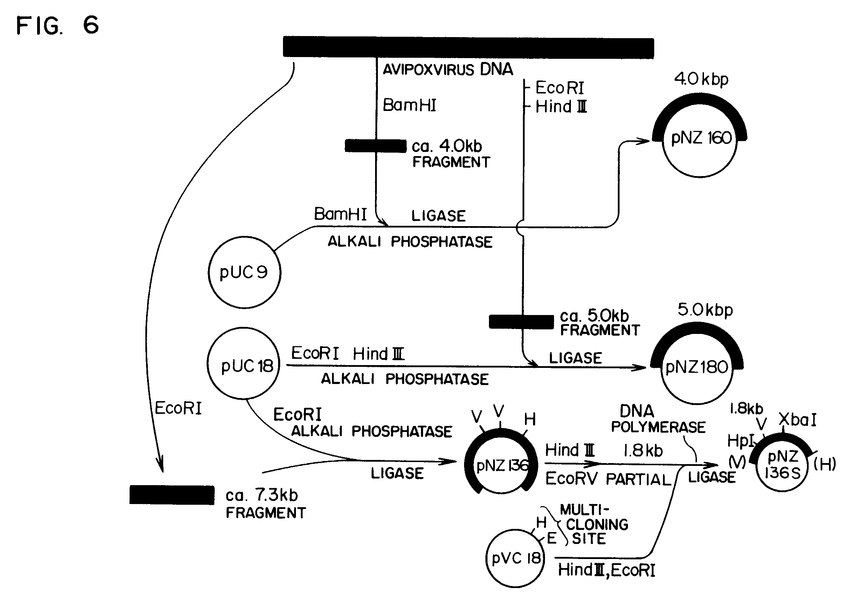

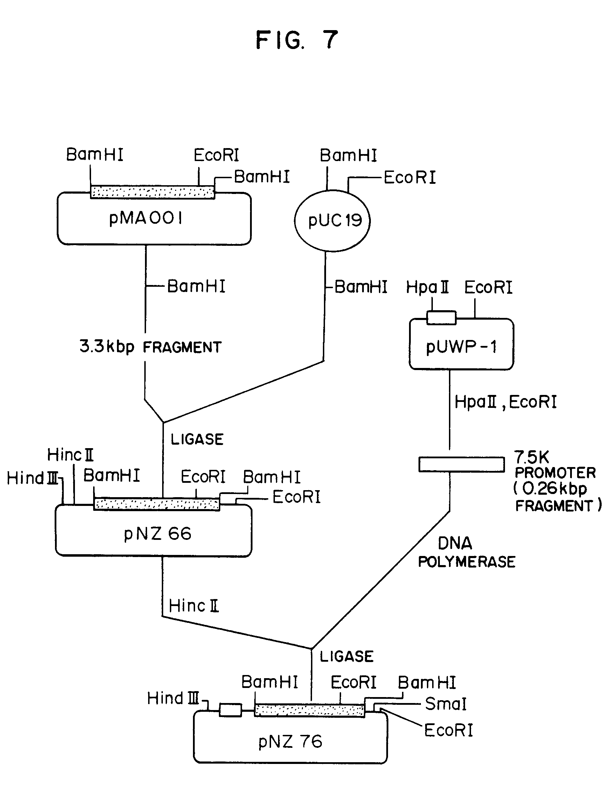

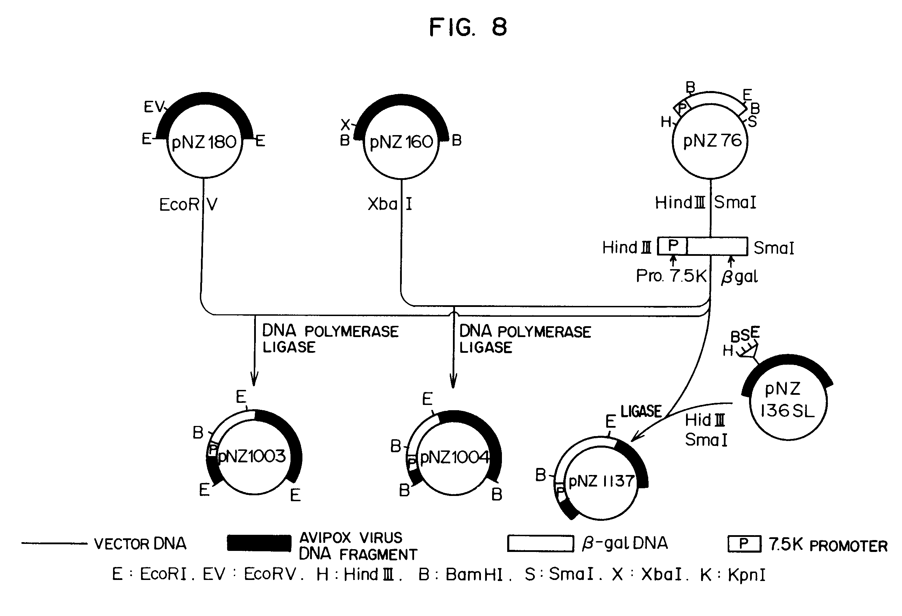

Figures 1, 2 and 3 show procedures for construction of recombinant Avipoxvirus. Figure 4 shows an amino acid sequence and base sequence of HN gene from NDV. Figure 5 shows an example of a DNA region non-essential to proliferation of Avipoxvirus. Figure 6 shows a procedure for construction of a plasmid vector containing at least a part of the DNA region non-essential to proliferation. Figure 6 illustrates procedures for construction of hybrid plasmid (A) referred to in the present invention. Figure 7 illustrates procedures for construction of hybrid plasmid (B). Figure 8 illustrates procedures for construction of hybrid plasmid (C).

### Detailed Description of the Preferred Embodiments

Any virus is usable as the virus employed to insert NDV-derived cDNA in the present invention as far as it is classified into the genus Avipoxvirus but preferred are those capable of growing in cells of fowls such as chicken, turkey, duck, etc. Specific examples include Fowlpoxvirus such as ATCC VR-251, ATCC VR-250, ATCC VR-229, ATCC VR-249, ATCC VR-288, Nishigawara strain, Shisui strain, etc.; and those akin to Fowlpoxvirus and used to avian live vaccine strain such as NP strain (chick embryo habituated dovepoxvirus Nakano strain), etc. These strains are all commercially available and easily accessible.

Further, cDNA coding for NDV-derived HN can be prepared using, for example, D-26 strain of NDV or Beaudette C strain. cDNA prepared from, for example, D-26 strain described above includes two kinds, one of which is composed of 1746 base pairs from 112th to 1857th and another is composed of 1848 base pairs from 112th to 1959th, among the base sequence shown in Figure 4. A difference in both cDNAs lies in 1747th base in Figure 4 (base marked with * in Figure 4) wherein, in the case of long cDNA, the base is C as shown in the figure but is substituted on T in the case of short cDNA and thus constitutes a translation termination codon (TAA). For information, cDNA used in the examples is short one.

Further cDNA prepared from Beaudette C strain is composed of 1731 base pairs [N. Miller, J. Gen. Virol., 67, 1917-1927 (1986)] and has a structure in which the base sequence are substituted at portions amounted to 170 in short cDNA prepared from D-26 strain.

As such, base sequences of cDNA encoding NDV-derived HN gene are not necessarily identical but in the present invention, cDNA may be modified one (namely, cDNA in which the base sequence is substituted, inserted or deleted), within such a range that cDNAs have substantially the same function as in the 3 kinds of cDNAs described above. Of course, insofar as they have substantially the same function, they may also be modified to such an extent that amino acid sequences are different.

In one way of performing the present invention, a first recombinant vector in which a DNA region non-essential to growth of Avipoxvirus has been inserted is firstly constructed. Preferred is a recombinant vector containing in the same region a promoter functioning in Avipoxvirus.

Specific examples of the DNA region non-essential to growth include regions which cause homologous recombination with EcoR I-Hind III fragment (about 5.0 kbp), BamH I fragment (about 4.0 Kbp), EcoR V-Hind III fragment (about 1.8 kbp), BamH I fragment (about 3.3 Kbp), Hind III fragment (about 5.2 Kbp), etc.

Such a DNA region non-essential to growth can be fixed, for example, by the following procedures. First, hybrid plasmid (A) having inserted therein an optional DNA fragment from Avipoxvirus genome and hybrid plasmid (B) having ligated DNA encoding an enzyme capable of being readily detected under control of a promoter are prepared. Next, a DNA fragment fully containing DNA coding for the promoter and the enzyme is prepared from hybrid plasmid (B). By inserting the DNA fragment into a virus DNA portion of hybrid plasmid (A), hybrid plasmid (C) is prepared. Then, hybrid plasmid (C) is transfected into a host cell previously infected with Avipoxvirus, whereby positive or negative formation of recombinant Avipoxvirus is confirmed.

Selection for examining as to if a recombinant Avipoxvirus is constructed by a series of procedures described above may be performed in a conventional manner. Where, for example, β-galactosidase gene is used as enzyme-coding DNA, agarose medium containing chlorophenol red-β-D-galactopyranoside (CPRG) is overlaid in layers after plaques are recognized on a medium for forming plaques of recombinant Avipoxvirus; plaques stained red by incubation at 37°C may thus be selected.

As such, in case that a recombinant Avipoxvirus is obtained using detection of enzyme activity as a means for selection, it is indicated that an Avipoxvirus-derived DNA fragment used for construction of hybrid plasmid (A) is a DNA region non-essential to growth.

The DNA having a promoter function as used in the present invention may be any DNA having any base sequence as long as it can effectively function as a promoter in the transcription system possessed by Avipoxvirus, irrespective of synthesized or naturally occurring DNA. Needless to say, promoters intrinsic to Avipoxvirus such as a promoter of Avipoxvirus gene coding for thymidine kinase are usable and, even DNA derived from virus other than Avipoxvirus or DNA derived from eucaryote or procaryote can be naturally used in the present invention, as far as it meets the requirement described above.

Concrete examples of these promoters include promoters of vaccinia virus illustrated in Journal of Virology, September 1984, 662-669, specifically, a promoter of vaccinia virus gene coding for 7.5 K polypeptide, a promoter of vaccinia virus gene coding for 19 K polypeptide, a promoter of vaccinia virus gene coding for 42 K polypeptide, a promoter of vaccinia virus gene coding for thymidine kinase polypeptide, a promoter of vaccinia virus gene coding for 28 K polypeptide, etc. The promoter may be complete or partly modified as long as it retains the function.

A first recombinant vector may be constructed in a conventional manner. For example, after inserting a DNA fragment non-essential to growth of Avipoxvirus into an appropriate vector, a promoter added with a restriction enzyme cleavage sequence may be incorporated at the downstream thereof, if necessary and desired, utilizing restriction enzyme cleavage sites present in the fragment.

Specific examples of the vector used herein include a plasmid such as pBR 322, pBR 325, pBR 327, pBR 328, pUC 7, pUC 8, pUC 9, pUC 19, etc.; a phage such as ! phage, M13 phage, etc.; a cosmid such as pHC 79 (Gene, 11, 291, 1980) and the like.

A second recombinant vector may be constructed by inserting a NDV HN-coding region into the promoter of the first recombinant vector at the downstream thereof. Insertion may also be effected in a conventional manner. For example, NDV-derived cDNA fragment may be inserted, utilizing a restriction enzyme cleavage site previously provided at the downstream of the promoter of the first vector.

Upon construction of these first and second recombinant vectors, the Escherichia coli system in which genetic manipulations are easy may be used. The plasmid vector used is not particularly limited as far as it is suited for purposes.

Next, the second recombinant vector may be transfected into animal culture cells previously infected with Avipoxvirus to cause homologous recombination between the vector DNA and the virus genome DNA, whereby a recombinant Avipoxvirus is constructed. The animal culture cells as used herein may be any cells insofar as Avipoxvirus can grow there. Specific examples are chick-derived culture cells such as chick embryo fibroblasts, etc. Furthermore, chick chorioallantoic membrane is also naturally included in the category of host cell.

Construction of the recombinant Avipoxvirus may be carried out in a conventional manner. The second recombinant vector treated by the calcium phosphate coprecipitation method is transfected into host cells infected with Avipoxvirus according to, for example, D.M. Glover, DNA Cloning, volume II, a practical approach, pp. 191-211, IRL Press, Oxford, Washington. The thus obtained virus mass containing a recombinant virus is infected to host cells cultured on Eagle's MEM medium. Plaques grown on the medium may be made recombinant virus candidate strains. For selection of a virus having inserted therein a DNA fragment coding for NDV HN from these candidate strains, the candidate strains may be purified by a hybridization method using the DNA as a probe and immunoassay using an NDV antibody is then applied.

Therefore, in accordance with the present invention, there is provided a recombinant Avipoxvirus which is utilizable as a live vaccine for fowls and in which NDV-derived antigen-coding cDNA has been inserted into a genome region non-essential to proliferation of Avipoxvirus, preferably together with a DNA having promoter functions, in the form capable of expression.

Embodiments of the present invention will be described in more detail with reference to the examples and the reference examples below. Reference Example 1 (identification of non-essential DNA region)

### (1) Construction of Avipoxvirus genome DNA:

Avipoxvirus NP strain (chick embryo habituated dovepoxvirus Nakano strain, manufactured by Japan Pharmacy Co., Ltd.) was inoculated on chick embryo fibloblasts cultured in a 75 cm² culture flask in 1 p.f.u./cell. After culturing in an incubator at 37°C in 5% CO₂ for 2 hours, 15 ml of Eagle's MEM medium supplemented with 10% Tryptose phosphate broth (Difco Co., Ltd.) and 0.03% L-glutamine was added followed by incubation in an incubator for 4 days at 37°C in 5% CO₂. Then the culture supernatant was centrifuged at 3000 r.p.m for 10 minutes to recover the supernatant. The supernatant was centrifuged at 25000 r.p.m. (about 98000 x g) for an hour and the precipitates were recovered. The precipitates were suspended in a DNase in a reaction buffer (50 mM Tris, pH 7.5, 1 mM MgCl₂) of a 1/10 volume of the culture supernatant and DNase I (Boehringer Mannheim Co., Ltd.) was added to the suspension in 9 »g/ml followed by reacting at 37°C for 30 minutes. After the reaction, 25 mM EDTA.2Na was added and the mixture was allowed to stand at room temperature for 30 minutes. Thereafter, 500 »g/ml of proteinase K (Boehringer Mannheim Co., Ltd.) and sodium dodecyl sulfate (SDS) were added to the mixture in a 1% concentration followed by reacting at 37°C overnight. After gently treating with phenol-chloroform, the reaction mixture was precipitated with ethanol to give 0.5 »g of virus DNA.

### (2) Construction of hybrid plasmid (A) containing Avipoxvirus genome fragment (cf. Figure 6)

### (a) Construction of plasmid (pNZ 180) containing about 5.0 Kb EcoR I-Hind III fragment of DNA of Avipoxvirus NP strain:

After digesting 2 »g of pUC 18 (manufactured by Pharmacia Inc.) with EcoR I and Hind III, extraction was performed with phenol-chloroform (1 : 1) to recover pUC 18 cleaved by precipitation with ethanol. 5′-End phosphate was removed by treating with alkali phosphatase. After extraction again with phenol-chloroform, DNA was recovered by precipitation with ethanol. The cleaved pUC 18, 0.2 »g, and the digestion product of 1 »g of purified Avipoxvirus (NP strain) DNA with EcoR I and Hind III were ligated with each other. Competent E. coli JM 103 was transformed and cultured at 37°C for 15 hours in LB agar medium supplemented with 0.003% of 5-bromo-4-chloro-3-indolyl-β-D-galactopyranoside, 0.03 mM of isopropyl-β-D-galactopyranoside and 40 »g/ml of ampicillin. White colony out of the transformed E. coli grown on agar medium was cultured at 37°C for 15 hours in LB liquid medium added with 40 »g/ml of ampicillin and plasmid was extracted by the method of Birnboim and Doly [Nucleic Acid Research, 7, 1513 (1979)]. After digesting with EcoR I and Hind IIID, a hybrid plasmid having a fragment of the same length as that of the original Avipoxvirus DNA EcoR I-Hind III fragment was detected by 0.6% agarose electrophoresis, which was named pNZ 180. A restriction enzyme map of the about 5.0 Kb EcoR I-Hind III fragment is shown in Fig. 5(a). pNZ 180 corresponds to hybrid plasmid (A).

### (b) Construction of plasmid (pNZ 160) containing about 4.0 Kb BamH I fragment of DNA from Avipoxvirus NP strain:

A hybrid plasmid was obtained in a manner similar to (a) except that vector pUC 18 used in (a) was changed to pUC 9 and about 4.0 Kb BamH I fragment of NP strain DNA was used in lieu of the EcoR I-Hind III fragment. This hybrid plasmid was named pNZ 160. A restriction enzyme map of about 4.0 Kb BamH I fragment is shown in Fig. 5(b) and pNZ 160 corresponds to hybrid plasmid (A).

### (c) Construction of plasmid (pNZ 136S) containing about 1.8 Kb EcoR V-Hind III fragment of DNA from Avipoxvirus NP strain:

A hybrid plasmid was obtained in a manner similar to (a) except that about 7.3 Kb EcoR I fragment of NP strain DNA wad used in lieu of about 5.0 Kb EcoR I-Hind III fragment used in (a). This hybrid plasmid gas named pNZ 136. Furthermore, after pNZ 136 was digested with Hind III, the digestion product was partially digested with EcoR V. The partial digestion product was subjected to 0.8% agarose electrophoresis to recover about 1.8 Kb EcoR V-Hind III fragment composed of about 0.6 Kb EcoR V-EcoR V fragment and about 1.2 Kb EcoR V-Hind III fragment adjacent to each other.

On the other hand, after pUC 18 was digested with Hind III and EcoR I, extraction was performed with phenol : chloroform (1 : 1) and the extract was precipitated with ethanol to recover cleaved pUC 18. The cleaved pUC 18 and about 1.8 Kb EcoR V-Hind III fragment recovered from agarose gel were blunted with DNA polymerase followed by ligation with ligase. Competent E. coli JM 103 was transformed and the transformants were selected on ampicillin-supplemented LB agar medium to give a plasmid containing about 1.8 Kb EcoR V-Hind III fragment. The plasmid was named pNZ 1365.

A restriction enzyme map of about 1.8 kb EcoR V-Hind III fragment is shown in Fig. 5(e) and pNZ 136S corresponds to hybrid plasmid (A).

### (3) Construction of a second hybrid plasmid (B) (pNZ 76) ligated with a promoter and DNA coding for readily detectable enzyme under its control (cf. Fig. 7)

A plasmid having inserted about 0.26 kbp of Sal I-Rsa I fragment containing a promoter of DNA coding for 7.5 K dalton peptide of vaccinia virus WR strain [Cell, 125, 805-813 (1981)] into Sal I-Sma I portion of pUC 9 was named pUWP-1.

After digesting 10 »g of pMA 001 [Shirakawa et al., Gene, 28, 127 (1984)] with BamH I, β-galactosidase gene (about 3.3 kbp) was recovered in a manner similar to (2). On the other hand, after digesting 0.3 »g of pUC 19 with BamH I, extraction was performed with phenol-chloroform and recovery was made through precipitation with ethanol. By ligation with the β-galactosidase gene prepared as described above, hybrid plasmid pNZ 66 was constructed.

On the other hand, 40 »g of pUWP-1 was digested with Hpa II and EcoR I and, a fragment of about 0.26 kbp containing 7.5 K promoter was separated by 1.5% low melting point agarose electrophoresis (70 volts, 6 hours) and DNA was recovered by operation similar to (2). The cohesive end of this DNA fragment was made the blunt end by DNA polymerase. After 0.3 »g of pNZ 66 was digested with Hinc II, extraction was performed with phenol-chloroform and recovery was made through precipitation with ethanol. By ligation with about 0.26 kbp of the 7.5 K promoter gene described above, a hybrid plasmid was obtained and named pNZ 76. pNZ 76 corresponds to hybrid plasmid (B).

### (4) Construction of hybrid plasmid (C) from hybrid plasmid (A) and hybrid plasmid (B) (cf. Fig. 8)

### (a) Construction of hybrid plasmid (pNZ 1003) having inserted a ligation fragment of vaccinia virus 7.5 K promoter and β-galactosidase DNA into EcoR V site of pNZ 180

After digesting 10 »g of pNZ 76 with Hind III and Sma I, a fragment of about 3.6 kbp was separated by 0.7% low melting point agarose electrophoresis (40 volts, 20 hours). After the DNA fragment was confirmed by staining with ethidium bromide, gel was excised, treated with phenol and precipitated with ethanol to recover the DNA fragment.

On the other hand, 1 »g of pNZ 180 was digested with EcoR V, extracted with phenol-chloroform and precipitated with ethanol to recover the same. The cleaved pNZ 180 DNA, 0.3 »g, was mixed with about 0.4 »g of the aforesaid about 3.6 kbp fragment (ligation fragment of 7.5 K promoter DNA and β-galactosidase gene) and the cohesive end was made the blunt end by DNA polymerase. After extracting with phenol-chloroform, the DNA was recovered. The recovered DNA was ligated with ligase. Competent E. coli JM 103 strain was transformed and allowed to grow at 37°C for 15 hours in LB agar medium supplemented with 40 »g/ml of ampicillin. A plasmid was recovered from E. coli grown in a manner similar to (2) and digested with BamH I. A hybrid plasmid containing a β-galactosidase gene fragment (about 3.3 kbp) was selected by 0.5% agarose electrophoresis, which was named pNZ 1003.

### (b) Construction of hybrid plasmid (pNZ 1004) having inserted a ligation fragment of vaccinia virus 7.5 K promoter and β-galactosidase DNA into Xba I site of pNZ 160

A hybrid plasmid containing a β-galactosidase gene fragment (about 3.3 kbp) was selected in a manner similar to (a) except that pNZ 180 and restriction enzyme EcoR V used in (a) were changed to pNZ 160 and Xba I, respectively. This hybrid plasmid was named pNZ 1004.

### (c) Construction of hybrid plasmid (pNZ 1137) having inserted a ligation fragment of vaccinia virus 7.5 K promoter and β-galactosidase DNA into EcoR V site of pNZ 136S

After digesting pNZ 136S with EcoR V, EcoR I linker (manufactured by Takara Shuzo Co., Ltd.) was linked to the digestion product with ligase. By extraction with phenol-chloroform and precipitation with ethanol, DNA was recovered. After the recovered DNA was likewise digested with EcoR I and Xba I, about 0.2 Kb EcoR I-Xba I fragment was recovered from 1.5% agarose gel. After digesting pNZ 136S with EcoR V, Hind III linker (manufactured by Takara Shuzo Co., Ltd.) was also linked to the digestion product with ligase. By extraction with phenol-chloroform and precipitation with ethanol, DNA was recovered. After the recovered DNA was digested with Hind III and Xba I, about 4.3 Kb Hind III-Xba I fragment was recovered from 0.8% agarose gel. Further after digesting pUC 18 with Hind III and EcoR I, 51 bp fragment of polylinker portion was recovered from 2.0% agarose gel. The about 0.2 Kb EcoR I-Xba I fragment, about 4.3 Kb Hind III-Xba I fragment and 51 bp Hind III-EcoR I fragment thus obtained were ligated with each other by ligase. A plasmid was extracted in a manner similar to Example 1 (4). A plasmid in which 3 fragments had been ligated was detected and this was named pNZ 136SL.

On the other hand, after digesting pNZ 76 with Hind III and Sma I, about 3.6 Kb fragment was recovered in a manner similar to (2). The pNZ 136SL described above was digested with Hind III and Sma I. By extraction with phenol-chloroform and precipitation with ethanol, recovery was performed. Both were ligated with each other by ligase and competent E. coli JM 103 strain was transformed. Subsequently in a manner similar to (2), a hybrid plasmid was selected and this was named pNZ 1137.

### (5) Construction of a recombinant Avipoxvirus:

Avipoxvirus NP strain was inoculated on chick embryo fibloblasts cultured in a 25 cm² culture flask in 0.05 p.f.u./cell. The hybrid plasmid, 50 »g, obtained in (4) was dissolved in 2.2 ml of sterilized water and, 2.5 ml of a mixture of 1% HEPES (manufactured by GIBCO Co., Ltd.) and 0.6% sodium chloride and 50 »l of buffer solution obtained by mixing 70 mM disodium hydrogenphosphate 12 hydrate and 70 mM disodium hydrogenphosphate 2 hydrate in an equimolar amount were mixed with the solution to prepare an aqueous solution. The aqueous solution was transferred to a 15 ml tube (manufactured by Falcon Co., Ltd.) and 300 »l of 2 M calcium chloride aqueous solution was dropwise added thereto while agitating with a stirrer to form DNA-calcium phosphate coprecipitates. Forty five minutes after the inoculation of virus, 0.5 ml of the coprecipitates were dropwise added to the infected chick embryo fibloblasts. After settling in an incubator at 37°C in 5% CO₂ for 30 minutes, 4.5 ml of Eagle's MEM medium supplemented with 5% bovine fetal serum, 0.03% L-glutamine and 10% Tryptose phosphate broth was added thereto. Three hours after, the culture supernatant was exchanged and cultured in an incubator for 3 days at 37°C in 5% CO₂. The system including culture cells was frozen and thawed 3 times to give a solution of virus containing recombinant.

### (6) Selection of a recombinant by chlorophenol red-β-D-galactopyranoside

The virus solution obtained in (5) was inoculated on chick embryo fibloblasts cultured in a 10 cm Petri dish. Two hours after, 10 ml of phenolred-free Eagle's MEM medium supplemented with 0.8% Bacto agar (manufactured by Difco Co., Ltd.), 5% bovine fetal serum, 0.03% L-glutamine and 10% Tryptose phosphate broth was put thereon in layers followed by incubation in an incubator at 37°C in 5% CO₂ for 3 days. On the medium was put 10 ml of phenol red-free Eagle's MEM medium supplemented with 0.8% Bacto agar, 0.03% L-glutamine, 10% Tryptose phosphate broth and 0.03% chlorophenol red-β-D-galactopyranoside (Boehringer Mannheim) in layers followed by incubation in an incubator at 37°C in 5% CO₂ for 6 hours. Recombinant virus expresses β-galactosidase and changes chlorophenol red-β-galactopyranoside to red so that both agar and cells around the recombinant plaque became red and could be easily distinguished over non-recombinant. The recombinant formed from this red plaque was isolated with a sterilized Pasteur pipette. Each recombinant was named as shown in Table 1.

**Table 1**

| Parent Strain | Hybrid Plasmid (C) | Recombinant |
|---|---|---|
| Avipoxvirus NP strain | pNZ 1003 | fNZ 1003 |
| Avipoxvirus NP strain | pNZ 1004 | fNZ 1004 |
| Avipoxvirus NP strain | pNZ 1137 | fNZ 1137 |

### (7) Analysis of genome DNA of the recombinant Avipoxvirus:

After each Avipoxvirus obtained in (6) was subjected to plaque purification, the Avipoxvirus was inoculated on chick embryo fibloblasts cultured in a 10 cm Petri dish in 1 p.f.u./cell. Subsequently in a manner similar to (1), virus DNA was isolated. After 2 »g of each recombinant virus DNA was digested with BamH I or Hind III, fragments were separated by 0.5% agarose electrophoresis (25V, 20 hours). After the DNA was transferred to a nylon membrane by the Southern's method [Journal of Molecular Biology, 98, 503 (1975)], the DNA was immobilized onto the nylon membrane at 80°C under reduced pressure. After treating with 4-fold SET-[0.6 M NaCl, 0.08 M Tris HCl (pH 7.8), 4 mM EDTA]-10-fold Denhardt-0.1% SDS at 68°C for 2 hours, β-galactosidase labelled with ³²P by nick translation was hybridized with 4-fold SET-10-fold Denhardt-0.1% SDS-modified salmon sperm DNA at 68°C for 14 hours. After washing and drying, a nylon membrane was put on an X ray film to perform autoradiography, whereby the presence of bands was confirmed. It was confirmed that these recombinant viruses contained the β-galactosidase genes in definite locations.

### (8) Identification of non-essential DNA region

From the foregoing results, it will be understood that the EcoR I-Hind III fragment (about 5.0 kbp) used for construction of pNZ 180, BamH I fragment (about 4.0 kbp) used for construction of pNZ 160 and EcoR V-Hind III fragment (about 1.8 kbp) are regions non-essential to proliferation of virus.

In a manner similar to the above, it has also been confirmed that BamH I fragment (about 3.3 kbp) and Hind III fragment (about 5.2 kbp) of DNA from the NP strain are also regions non-essential to proliferation of virus. A restriction enzyme map of these fragments is as shown in Figure 5.

Furthermore, hybrid plasmids pNZ 1003, pNZ 1004 and pNZ 1137 constructed in (4) all contain the DNA region non-essential to growth of Avipoxvirus and can thus be utilized as a first recombinant vector in the present invention.

### Example 1 Construction of a second recombinant vector (pNZ 2003) containing HN gene DNA of NDV (cf. Fig. 1)

### (1) Construction of hybrid phage (mp10-mpa) containing adaptor:

After digesting 0.1 »g of M13-mp10 RF DNA (manufactured by Amersham Co., Ltd.) with BamH I and Xba I, the digestion product was extracted with phenol and chloroform (1 : 1). By precipitation with ethanol, cleaved M13-mp10 RF DNA was recovered.

On the other hand, an adaptor composed of 40 bp oligodeoxyribonucleotide having a base sequence described below was chemically synthesized using Genet AIII (manufactured by Nippon Zeon Co., Ltd.). This adaptor was mixed with the cleaved M13-mp10 RF DNA, ligated by ligase and transduced into competent E. coli TG-1 strain in a conventional manner (Methods in Enzymology, vol. 101). Incubation was carried out in 2 x YT agar medium supplemented with 0.003% 5-bromo-4-chloro-3-indolyl-β-D-galactopyranoside and 0.03 mM isopropyl-β-D-galactopyranoside for 12 hours at 37°C. Among plaques formed, phage RF DNA was recovered from white plaque. This DNA was cleaved with Bgℓ II. By 0.8% agarose gel electrophoresis, a hybrid phage having inserted therein adaptor DNA was selected and this was named mp10-mpa.

Bgℓ II                                    Xho  I

Xba I                              Kpn  I              BamH  I

CTAGATCTAAGGAGGAAAAAATTATGGTACCTCGAGCTCG

    TAGATTCCTCCTTTTTTAATACCATGGAGCTCGAGCCTAG

### (2) Construction of hybrid phage (mp10-HN 115) containing Ava II (about 1.15 kbp) fragment of HN gene DNA of NDV

Plasmid XLIII-10H containing NDV HN gene (cf., Virus Research, 7, 241-255 (1987)) was obtained from Assistant Professor Masao Kawakita, of Tokyo University, Department of Liberal Arts. A base sequence of this plasmid was analyzed by the dideoxy method using M13 phage and, it has been clarified by contrasting to the base sequence of known HN gene [J. Gen. Virol., 67, 1917-1927 (1986)] that cDNA of HN gene is 1746 base pairs from 112th to 1857th shown in Fig. 4. This clarification was made by Assistant Professor Kawakita.

The amino acids for which the cDNA obtained herein codes had 90% or more homology to amino acids of HN from the Beaudette strain [N. Miller, J. Gen. Virol., 67, 1917-1927 (1986)] and B₁ strain [E. Jorgensen, Virology, 156, 12-24 (1987)].

After digesting 4 »g of plasmid XLIII-10H with Ava II, the digestion product was subjected to 1.5% agarose gel electrophoresis. About 1.15 kbp DNA fragment was extracted from agarose gel and precipitation was carried out with ethanol to recover Ava II fragment of HN gene DNA.

After digesting hybrid phage mp10-mpa RF DNA constructed in (1) with Kpn I, extraction was performed with phenol-chloroform and precipitation with ethanol thereby to recover the cleaved mp10-mpa.

The cleaved mp10-mpa RF DNA, 0.1 »g, was mixed with about 0.2 »g of the Ava II fragment (about 1.15 kbp) of HN gene DNA described above and the cohesive end was made the blunt end by DNA polymerase. After extracting with phenol-chloroform, the DNA was recovered by precipitation with ethanol. The recovered DNA was ligated with ligase and transduced into competent E. coli TG-1 strain, which was allowed to grow in 2 x YT agar medium at 37°C for 12 hour. Phage RF DNA was recovered from the formed plaque. After cleaving with Xba I and Xho I, a hybrid phage containing Ava II fragment (about 1.15 kbp) of HN gene DNA was selected by 0.8% agarose electrophoresis. The hybrid phage was named mp10-HN 115 RF.

### (3) Construction of hybrid phage (mp10-HN 180) containing HN gene DNA of NDV

Plasmid XLIII-10H, 4 »g, was digested with Aat II and Xho I and about 1.4 kbp Aat II-Xho I fragment of HN gene DNA was recovered in a manner similar to (2).

On the other hand, after hybrid phage mp10-HN 115 RF DNA was digested with Aat II and Xho I, the digestion product was subjected to 0.6% agarose electrophoresis to recover Aat II-Xho I fragment (about 7.7 kbp) of mp10-HN 115.

The Aat II-Xho I fragment (about 7.7 kbp) of HN gene was ligated with the Aat II-Xho I fragment (about 1.4 kbp) of mp10-HN 115 by ligase and phage RF DNA was recovered by operations similar to (2). Phage RF DNA was cleaved with Xho I and Bgℓ II and, hybrid phage RF DNA containing about 1.8 kbp HN gene DNA fragment was selected by 0.8% agarose electrophoresis, which was named mp10-HN 180 RF.

### (4) Construction of a second recombinant vector (pNZ 2003) from the first recombinant vector pNZ 1003 and hybrid phage mp10-HN 180 RF DNA:

The first recombinant vector pNZ 1003 constructed in Reference 1 (4) was partially digested with BamH I and the product was subjected to 0.6% agarose electrophoresis. Following procedures similar to (2), about 7.6 kbp BamH I fragment was recovered. On the other hand, hybrid phage mp10-HN 180 RF DNA constructed in (3) was digested with BamH I and Bgℓ II and the product was subjected to 0.8% agarose electrophoresis. Then, BamH I-Bgℓ II fragment (about 1.8 kbp) containing HN gene DNA was recovered.

The BamH I fragment of about 7.6 kbp obtained by partial digestion of pNZ 1003 with BamH I was mixed with the about 1.8 kbp BamH I-Bgℓ II fragment containing HN gene DNA. The mixture was ligated with ligase and competent E. coli TG-1 strain was transformed, which was allowed to grow on LB agar medium supplemented with 40 »g/ml of ampicillin at 37°C for 15 hours. A plasmid was recovered from the grown E. coli in a manner similar to Reference Example 1 (2). The plasmid was digested with BamH I and a recombinant vector containing a fragment (about 3.1 kbp) having ligated therewith 7.5 k promoter and HN gene was selected by 0.8% agarose electrophoresis. This vector was named pNZ 2003.

pNZ 2003 corresponds to a second recombinant vector in the present invention.

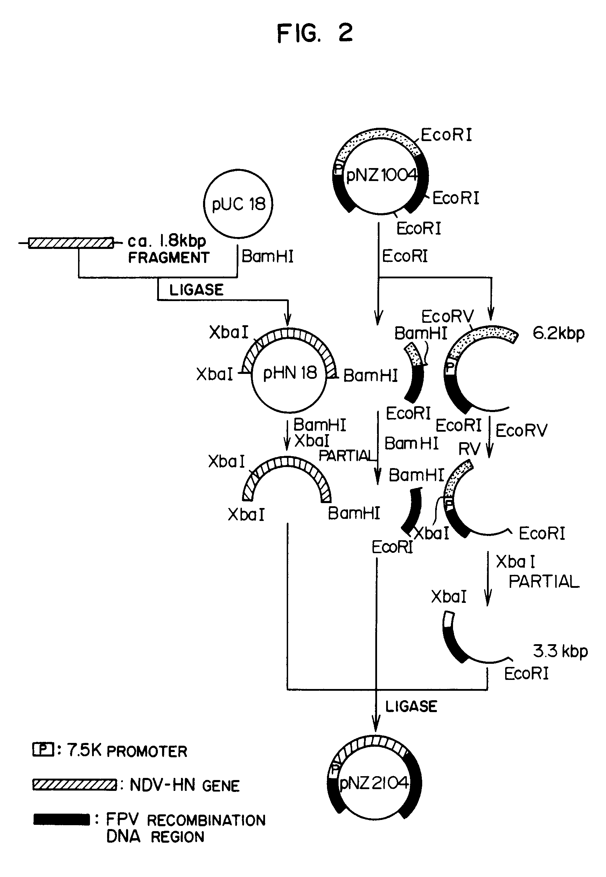

### Example 2 Construction of a second recombinant vector (pNZ 2104) containing NDV HN gene DNA (cf. Fig. 2)

### (1) Construction of hybrid plasmid pHN 18 from hybrid phage mp10-HN 180 RF DNA

pUC 18 was digested with BamH I. After extraction with phenol-chloroform, cleaved pUC 18 was recovered by precipitation with ethanol. This fragment and the about 1.8 kbp BamH I-Bgℓ II fragment containing HN gene DNA obtained in Example 1 (4) were ligated by ligase and competent E. coli TG-1 strain was transformed. A plasmid was extracted in a manner similar to Reference Example 1 (2). After digesting with BamH I and Xba I, a hybrid plasmid containing HN gene was detected by 0.8% agarose electrophoresis and this was named pHN 18.

### (2) Construction of a second recombinant vector (pNZ 2104) from the first recombinant vector pNZ 1004 and hybrid plasmid pHN 18

The first recombinant vector pNZ 1004 constructed in Reference 1 (4) was digested with EcoR I and the product was subjected to 0.6% agarose electrophoresis, whereby about 6.2 kbp DNA fragment and about 3.5 kbp DNA fragment were independently recovered. After the about 6.2 kbp EcoR I fragment was digested with EcoR V and further partially digested with Xba I, the digestion product was subjected to 0.8% agarose electrophoresis to recover about 3.3 kbp Xba I-EcoR I fragment containing 7.5 k promoter. Further the 3.5 kbp EcoR I fragment was further digested with BamH I and then subjected to 0.8% agarose electrophoresis to recover BamH I-EcoR I fragment of about 3.3 kbp.

On the other hand, hybrid plasmid pHN 18 constructed in (1) was digested with BamH I and then partially digested with Xba I. The digestion product was subjected to 0.8% agarose electrophoresis to recover BamH I-Xba I fragment containing HN gene DNA (about 1.8 kbp). The about 3.3 kbp Xba I-EcoR I fragment obtained from pNZ 1004 described above, the about 3.3 kbp Xba I-EcoR I fragment and the about 1.8 kbp BamH I-Xba I fragment containing HN gene DNA were mixed and ligated by ligase. In a manner similar to Example 1 (4), a recombinant vector containing a fragment (about 2.1 kbp) having ligated therewith 7.5 k promoter and HN gene was selected and named pNZ 2104. pNZ 2104 corresponds to a second recombinant vector in the present invention.

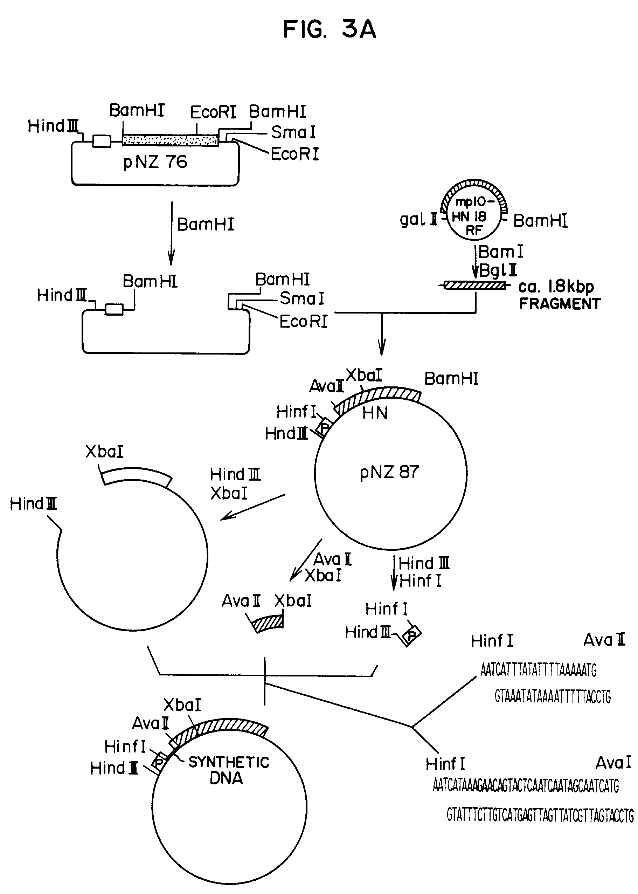

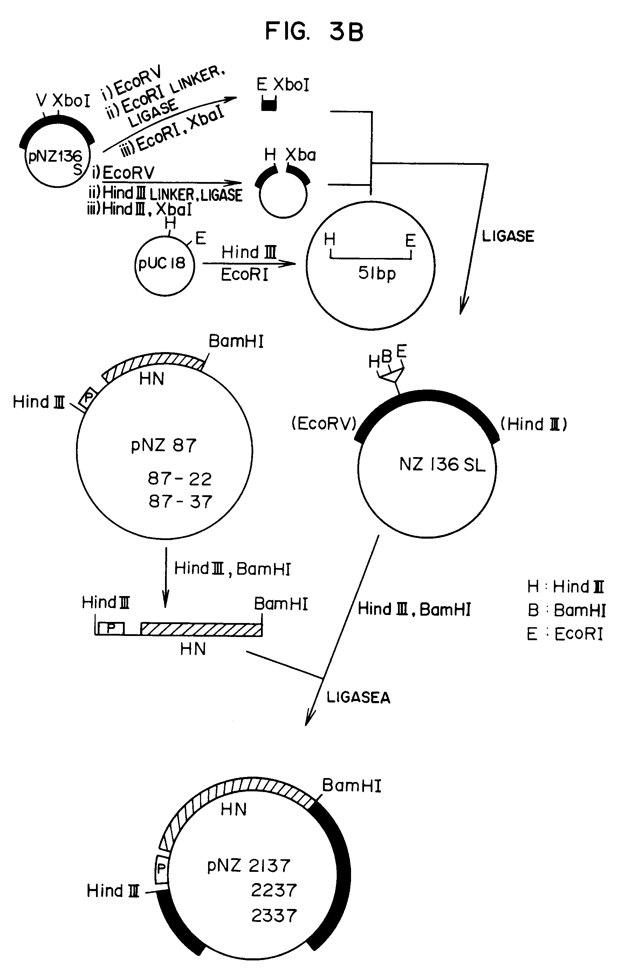

### Example 3 Construction of second recombinant vectors (pNZ 2137, pNZ 2237 and pNZ 2337) containing NDV HN gene DNA (cf. Fig. 3)

### (1) Construction of hybrid plasmid pNZ 87 having ligated therewith a promoter and NDV HN gene DNA under its control, from hybrid phage mp10-HN 180

pNZ 76 was digested with BamH I and about 2.9 kb fragment free from β-galactosidase gene was recovered from 0.8% agarose gel. On the other hand, hybrid phage mp10-HN 180 was digested with Bgℓ II and BamH I and NDV HN gene DNA fragment was then recovered from 0.8% agarose gel. Both were ligated by ligase and competent E. coli TG-1 was transformed. A plasmid was extracted and a hybrid plasmid containing HN gene was detected in a manner similar to Reference Example 1 (2). This was named pNz 87.

### (2) Construction of vaccinia virus 7.5 k promoter-bearing plasmids (pNZ 87-22 and pNZ 87-37) in which base sequence of HN gene up to translation initiation sequence has been changed

pNZ 87 was digested with Xba I and Hind III to recover about 4.3 kb of Xba I-Hind III fragment (fragment (1)). Likewise, pNZ 87 was digested with Xba I and Ava II to recover about 0.2 kb of Xba I-Ava II fragment (fragment (2)). Further pNZ 87 was digested with Hind III and Hinf I to recover about 0.2 kb of Hind III-Hinf I fragment (fragment (3)). To the fragments (1), (2) and (3) obtained herein was added synthetic DNA (4) (22 bp) shown below: 
Hinf I Ava II
AATCATTTATATTTTAAAAATG
GTAAATATAAAATTTTTACCTG
or synthetic DNA (5) (37 bp) shown below: 
Hinf I                                    Ava II
AATCATAAAGAACAGTACTCAATCAATAGCAATCATG
GTATTTCTTGTCATGAGTTAGTTATCGTTAGTACCTG
followed by ligation with ligase. Plasmids were extracted in a manner similar to Example 1 (4) and hybrid plasmids containing synthetic DNA (4) and (5), respectively, were detected. These plasmids were named pNZ 87-22 and pNZ 87-37.

### (3) Construction of second recombinant vectors (pNZ 2137, pNZ 2237 and pNZ 2337) from hybrid plasmids pNZ 87, pNZ 87-22 and pNZ 87-37 having ligated therewith pNZ 136SL corresponding to hybrid plasmid (A), promoter and HN gene DNA under control of the promoter

Hybrid plasmid pNZ 136SL constructed in Reference Example 1 (2)-(c) was digested with Hind III and BamH I. After extraction with phenol-chloroform, recovery was performed by precipitation with ethanol. On the other hand, after pNZ 87, pNZ 87-22 and pNZ 87-37 were digested with Hind III and BamH I, respectively, the digestion products were subjected to 0.8% agarose electrophoresis to recover about 2.1 kb Hind III-BamH I fragment containing vaccinia virus 7.5 k promoter and HN gene DNA. The former was ligated with each of the latter using ligase. Recombinant vectors bearing a fragment (about 2.1 kb) having ligated therewith 7.5 k promoter and HN gene were selected and named pNZ 2137, pNZ 2237 and pNZ 2337, respectively. They correspond to a second recombinant vector in the present invention.

### Example 4 Construction of a recombinant Avipoxvirus containing NDV HN gene

A solution of recombinant Avipoxvirus containing NDV HN gene was obtained in a manner similar to Reference Example 1 (5) except that the second recombinant vectors obtained in Examples 1, 2 and 3 were used in place of the hybrid plasmid used in Reference Example 1 (5).

### Example 5 Selection of recombinant by plaque hybridization

The virus solution obtained in Example 4 was inoculated on chick embryo fibloblasts cultured in a 10 cm Petri dish. Two hours after, 10 ml of Eagle's MEM medium supplemented with 0.8% Bacto agar, 5% bovine fetal serum, 0.03% L-glutamine and 10% Tryptose phosphate broth was overlaid in layers followed by incubation in an incubator at 37°C in 5% CO₂ for 3 days. On the medium was further overlaid in layers 10 ml of Eagle's MEM medium having the same composition as described above followed by incubation in an incubator at 37°C in 5% CO₂ for 3 days. Further 10 ml of Eagle's MEM medium supplemented with 0.8% Bacto agar, 0.03% L-glutamine, 10% Tryptose phosphate broth and 0.01% of neutral red was overlaid in layers followed by incubation in an incubator at 37°C in 5% CO₂ for 12 hours to stain infected cells.

The agar medium was withdrawn from the Petri dish. A sterilized nylon membrane was pushed onto the surface of cells kept at 4°C and remained on the bottom of the Petri dish to transfer the viruses thereon. After repeating a treatment with 0.5 M NaOH for 10 minutes and with 1 M Tris-hydrochloride buffer for 5 minutes 3 times, the system was treated with 1.5 M NaCl and 0.5 M Tris-hydrochloride buffer for 5 minutes. The system was saturated with 2-fold SSC [1-fold SSC, 0.15 M NaCl, 0.015 M C₃H₄(OH)(COONa)₃] and sintered at 80°C for 2 hours. The system was treated with 4-fold SET (0.6 M NaCl, 0.08 M Tris HCl, 4 mM EDTA, pH 7.8)-10-fold Denhardt-0.1% SDS at 68°C for 2 hours. 4-Fold SET-10-fold Denhardt-0.1% SDS-0.1% Na₄P₂O₇-50 »g/ml-modified salmon sperm DNA and cDNA coding for HN of NDV labeled with ³²P were hybridized by nick translation at 68°C for 14 hours. After washing, the nylon membrane was put on an X ray film, which was subjected to autoradiography to confirm the presence of a spot. The X ray film was overlaid on the agar kept at 4°C and a plaque coincided with the spot was identified to be a recombinant containing HN gene. The recombinant was isolated with a sterilized Pasteur pipette. Two plaques of this recombinant appeared per 10 Petri dishes of 10 cm in which approximately 500 plaques appeared (about 0.04%).

With respect to the thus obtained recombinant viruses, a recombinant virus obtained using pNZ 2003 was named fNZ 2003. Likewise, those obtained using pNZ 2104, pNZ 2137, pNZ 2237 and pNZ 2337 were named fNZ 2104, fNZ 2137, fNZ 2237 and fNZ 2337, respectively.

### Example 6 Purification of recombinant Avipoxvirus containing NDV HN gene

The plaque isolated in Example 5 was suspended in 1 ml of Eagle's MEM and 200 »l of the suspension was inoculated on chick embryo fibloblasts cultured in a 10 cm Petri dish. Two hours after, 10 ml of phenol red-free Eagle's MEM medium supplemented with 0.8% Bacto agar, 5% bovine fetal serum, 0.03% L-glutamine and 10% Tryptose phosphate broth was overlaid thereon in layers followed by incubation in an incubator at 37°C in 5% CO₂ for 3 days. On the medium was overlaid in layers 10 ml of phenol red-free Eagle's MEM medium having the same composition described above followed by incubation in an incubator at 37°C in 5% CO₂ for 3 days. On the medium was overlaid in layers 10 ml of phenol red-free Eagle's MEM medium supplemented with 0.8% Bacto agar, 0.03% L-glutamine and 10% Tryptose phosphate broth followed by incubation in an incubator at 37°C in 5% CO₂ for 6 hours. The recombinant plaque formed a spot on an X ray film as in Example 5.

The foregoing operations were repeated in a similar manner to again purify the recombinant.

As a result, 20 recombinant plaques (red plaque) (0.2%) appeared with fNZ 1004, per 10 Petri dishes of 10 cm in which approximately 500 plaques per dish appeared in Example 5; in the first plaque purification, 90 (15%) of the recombinant plaques appeared in 3 Petri dishes in which about 200 plaques per dish appeared. In the second plaque purification, almost all plaques were recombinants. Also with respect to fNZ 2104, fNZ 2137, fNZ 2237 and fNZ 2337, almost the same results were obtained.

### Example 7 Expression of recombinant in cell (fluorescent antibody technique)

Virus strains (fNZ 2003, fNZ 2104, fNZ 2137, fNZ 2237 and fNZ 2337) of the present invention showing m.o.i of 1.0 or 0.1 were inoculated on chick embryo fibloblasts cultured in a chamber slide for tissue culture. After culturing at 37°C in a 5% CO₂ incubator for 2 hours, Eagle's MEM medium supplemented with 10% Tryptose phosphate broth (Difco Co., Ltd.) and 0.03% L-glutamine was added. Thereafter, incubation was performed for 2 days at 37°C in a 5% CO₂ incubator and the medium was then withdrawn. After washing with PBS (phosphate buffered saline) and air drying, the system was treated with acetone at -20°C for 20 minutes to immobilise the cells. By examining in accordance with fluorescent antibody technique using as a primary antibody anti-NDV chick antibody and as a secondary antibody fluorescein isocyanate-bound anti-chick IgG antibody, it was confirmed that the recombinant Avipoxviruses had NDV HN protein productivity.

### Example 8 Expression of recombinant in cell (enzyme antibody technique)

The virus solution obtained in Example 6 was inoculated on chick embryo fibloblasts cultured in a 10 cm Petri dish. Two hours after, 10 ml of Eagle's MEM medium supplemented with 0.8% Bacto agar, 5% bovine fetal serum, 0.03% L-glutamine and 10% Tryptose phosphate broth was overlaid in layers followed by incubation in an incubator at 37°C in 5% CO₂ for 3 days. Furthermore, 10 ml of Eagle's MEM having the same composition as described above was overlaid in layers followed by incubation in an incubator at 37°C in 5% CO₂ for 3 days.

The agar medium was withdrawn from the Petri dish. A sterilized nylon membrane was pushed onto the surface of cells remained on the bottom of the Petri dish to transfer virus plaques and cells thereon. After treating with PBS (phosphate buffered saline) containing 2% skimmed milk for 30 minutes, anti-NDV chick antibody was reacted as a primary antibody at room temperature for an hour. After the reaction, the mixture was washed with PBS and biotinated anti-chick IgG antibody was reacted as a secondary antibody at room temperature for an hour. After the reaction, the mixture was washed with PBS and horse radish peroxidase-bound avidin-biotin complex was reacted at room temperature for 30 minutes. After the reaction, a color was formed with 8 mM Tris-hydrochloride buffer (pH 8.0) containing 0.05% 4-chloro-1-naphthol and 0.3% H₂O₂. By checking with the foregoing enzyme antibody method, it was confirmed that the recombinant Avipoxviruses had NDV HN protein productivity.

### Example 9

Recombinant virus was cultured in chick embryo fibroblast (CEF) in five flasks (150 cm²/flask) at 37°C for 48 hours. After recombinant virus propagated-CEF was subjected to freeze-thawing twice, a cell suspension was collected and centrifuged at 2,000 rpm for 15 minutes. To 130 ml of the supernatant were added 40 ml of 20 %(v/v) sodium glutamate solution and 30 ml of 10 %(v/v) saccharose solution. The mixture was put into 100 vials by 2 ml each. After preliminary freezing for 1.5 hours, freeze-drying was conducted for 30 hours under reduced pressure. One vial of vaccine was dissolved in 10 ml of physiological saline supplemented with 30 %(v/v) glycerol, and 0.01 ml/chick of the solution was inoculated into the right wing web of 7-day-old specific pathogen free (SPF) chick by the stick method. After inoculation, vaccination rash of chick was observed. At 2 weeks postvaccination hemagglutination-inhibition (HI) antibody of Newcastle disease virus (NDV) was measured. Virulent NDV Sato strain and virulent fowlpox virus Nishigahara strain were challenged at 2 and 3 weeks postvaccination respectively, whereby an immune effect of the vaccine was determined.

HI test of NDV was performed as described below. Twenty-five »l of NDV hemagglutination (HA) antigen containing 4 HA units were added to an equal volume of 2-fold serial serum dilution in microtiter plate. The plate were allowed to stand at room temperature for 10 minutes followed by mixing. Then 50 »l of 0.5 %(v/v) chicken red blood cell suspension was added to each well and the mixture was allowed to stand at room temperature for 45 minutes. HI titer was determined as the reciprocal of the highest dilution showing complete inhibition.

Effects of the vaccine are shown in Table 2. In the chick inoculated with the recombinant vaccine occurrence of skin swelling, that showed the reaction of chick with fowlpox virus vaccine, was noted as in ordinary fowlpox virus vaccine and no eruption was observed followed by challenge of fowlpox virus virulent strain at 3 weeks postvaccination. Furthermore, HI antibody of chick to NDV was detected and chick was resistant to challenge of virulent NDV strain at 2 weeks post-vaccination.

From these results, it is shown that a recombinant vaccine of the present invention is capable of immunizing chicks against fowlpox and NDV infection at the same time.

**Table 2**

| Group | Chick No. | State of Fowlpox | ND-HI Titer | | Challenge* | |
|---|---|---|---|---|---|---|
| | | | 2 Weeks | 3 Weeks | FPV | NDV |
| Vaccinated group | 1 | take | 1: 64 | 1:256 | - | - |
| | 2 | " | 1:128 | 1: 64 | - | - |
| | 3 | " | 1: 16 | 1: 32 | - | + |
| | 4 | " | 1: 16 | 1: 64 | - | - |
| | 5 | " | 1: 16 | 1: 64 | - | - |
| Intact control group | 1 | no pox | <1:2 | <1:2 | + | + |
| | 2 | " | <1:2 | <1:2 | + | + |
| | 3 | " | <1:2 | <1:2 | + | + |
| | 4 | " | <1:2 | <1:2 | + | + |
| | 5 | " | <1:2 | <1:2 | + | + |

| | | | | | | |
|---|---|---|---|---|---|---|
| * FPV + : Eruption is noted. (3 weeks) - : No eruption is noted. NDV + : Symptoms and death are noted. (2 weeks) - : Resistance to challenge is noted. | | | | | | |

## Claims (Claims for the following Contracting State(s): BE, DE, FR, GB, NL)

1. A vaccine for fowl which comprises a recombinant Avipoxvirus having the capability of inducing an anti-Newcastle Disease Virus protective response in a host and having inserted therein the whole or part of a nucleotide sequence coding for Newcastle Disease Virus-derived hemagglutinin neuraminidase, into a genome region non-essential to proliferation of Avipoxvirus.

2. A vaccine according to claim 1, wherein said nucleotide sequence is under control of a promoter.

3. A vaccine according to claim 1 or claim 2, wherein said nucleotide sequence encodes an amino acid sequence as shown in figure 4 or polypeptide having substantially the same immunogenicity as said amino acid sequence of figure 4.

4. A vaccine according to claim 1 or claim 2 wherein said nucleotide sequence has a base sequence as shown in figure 4 or base sequence coding for at least one epitope of hemagglutinin neuraminidase which has an ability to raise a neutralizing antibody against Newcastle Disease virus.

5. A vaccine according to any one of claims 1 to 4 wherein said genome region non-essential to proliferation of Avipoxvirus is selected from the group consisting of those which cause homologous recombination with an approximately 5.0 kbp EcoRI-HindIII fragment, an approximately 4.0 kbp BamHI fragment, an approximately 1.8 kbp EcoRV-HindIII fragment, an approximately 3.3 kbp BamHI fragment, or an approximately 5.2 kbp HindIII fragment of dovepoxvirus of the Nakano strain.

6. A vaccine comprising a live Nakano dovepoxvirus having inserted therein all or part of a nucleotide sequence coding for Newcastle Disease Virus-derived hemagglutinin neuraminidase in a genome region non-essential to proliferation of the Nakano dovepoxvirus virus.

7. A vaccine according to any one of claims 1 to 6 which is capable of giving to the fowl an anti-viral immunity against fowl pox virus and Newcastle Disease virus.

8. Use of a recombinant Avipoxvirus as defined in any one of claims 1 to 6 in the preparation of a vaccine for fowl.

9. Use of a recombinant Avipoxvirus as defined in any one of claims 1 to 6 in the preparation of a vaccine for fowl capable of giving the fowl an anti-viral immunity against fowl pox virus and Newcastle Disease virus.

## Patentansprüche (Patentansprüche für folgende(n) Vertragsstaat(en): BE, DE, FR, GB, NL)

1. Impfstoff für Geflügel, umfassend ein rekombinantes Vogelpockenvirus mit der Fähigkeit zur Induktion einer Schutzreaktion gegen das "Newcastle Disease"-Virus in einem Wirt, in den die gesamte oder ein Teil einer Nucleotidsequenz, die die vom "Newcastle Disease" -Virus stammende Hämagglutinin-Neuraminidase codiert, in einen Genombereich inseriert ist, der für die Vermehrung des Vogelpockenvirus nicht essentiell ist.

2. Impfstoff nach Anspruch 1, wobei die Nucleotidsequenz unter der Kontrolle eines Promotors steht.

3. Impfstoff nach Anspruch 1 oder 2, wobei die Nucleotidsequenz eine Aminosäuresequenz codiert, wie sie in Figur 4 gezeigt ist, oder ein Polypeptid, das im wesentlichen die gleiche Immunogenität wie die Aminosäuresequenz von Figur 4 aufweist.

4. Impfstoff nach Anspruch 1 oder 2, wobei die Nucleotidsequenz eine Basensequenz aufweist, wie sie in Fig. 4 gezeigt ist, oder eine Basensequenz, die mindestens ein Epitop der Hämagglutinin-Neuraminidase codiert, die die Bildung eines neutralisierenden Antikörpers gegen "Newcastle Disease"-Virus anregen kann.

5. Impfstoff nach einem der Ansprüche 1 bis 4, wobei der Genombereich, der für die Vermehrung des Vogelpockenvirus nicht essentiell ist, ausgewählt ist aus denen, die eine homologe Rekombination mit einem etwa 5,0 kbp großen EcoRI-HindIII-Fragment, einem etwa 4,0 kbp großen BamHI-Fragment, einem etwa 1,8 kbp großen EcoRV-HindIII-Fragment, einem etwa 3,3 kbp großen BamHI-Fragment oder einem etwa 5,2 kbp großen HindIII-Fragment des Taubenpockenvirus vom Nakano-Stamm verursachen.

6. Impfstoff, umfassend ein lebendes Nakano-Taubenpockenvirus, in das die vollständige oder ein Teil einer Nucleotidsequenz inseriert ist, die die von "Newcastle Disease"-Virus stammende Hämagglutinin-Neuraminidase codiert, in einen Genombereich, der für die Vermehrung des Nakano-Taubenpockenvirus nicht essentiell ist.

7. Impfstoff nach einem der Ansprüche 1 bis 6, der dem Geflügel eine antivirale Immunität gegen Geflügelpockenvirus und "Newcastle Disease"-Virus verleihen kann.

8. Verwendung eines rekombinanten Vogelpockenvirus gemäß der Definition nach einem der Ansprüche 1 bis 6 für die Herstellung eines Impfstoffs für Geflügel.

9. Verwendung eines rekombinanten Vogelpockenvirus gemäß der Definition in einem der Ansprüche 1 bis 6, für die Herstellung eines Impfstoffs für Geflügel, der dem Geflügel eine antivirale Immunität gegen Geflügelpockenvirus und "Newcastle Disease"-Virus verleihen kann.

## Revendications (Revendications pour l'(les) Etat(s) contractant(s) suivant(s): BE, DE, FR, GB, NL)

1. Vaccin destiné à des volailles, qui comprend un avipoxvirus recombinant qui est capable de provoquer chez un hôte une réponse protectrice contre le virus de la maladie de Newcastle et dans lequel est insérée la totalité ou une partie d'une séquence de nucléotides codant une hémagglutinine neuraminidase dérivée du virus de la maladie de Newcastle, dans une région du génome non-essentielle pour la prolifération de l'avipoxvirus.

2. Vaccin conforme à la revendication 1, dans lequel ladite séquence de nucléotides se trouve sous le contrôle d'un promoteur.

3. Vaccin conforme à la revendication 1 ou 2, dans lequel ladite séquence de nucléotides code la séquence d'acides aminés indiquée sur la Figure 4, ou un polypeptide présentant pratiquement les mêmes propriétés immunogènes que ladite séquence d'acides aminés de la Figure 4.

4. Vaccin conforme à la revendication 1 ou 2, dans lequel ladite séquence de nucléotides présente la séquence de bases indiquée sur la Figure 4 ou une séquence de bases codant au moins un épitope de l'hémagglutinine neuraminidase qui est capable de susciter un anticorps neutralisant contre le virus de la maladie de Newcastle.

5. Vaccin conforme à l'une des revendications 1 à 4, dans lequel ladite région du génome non-essentielle pour la prolifération de l'avipoxvirus est choisie dans l'ensemble que constituent celles qui provoquent une recombinaison par homologie avec un fragment EcoRI-HindIII d'environ 5,0 kb, un fragment BamHI d'environ 4,0 kb, un fragment EcoRV-HindIII d'environ 1,8 kb, un fragment BamHI d'environ 3,3 kb ou un fragment HindIII d'environ 5,2 kb, provenant du colombopoxvirus de souche Nakano.

6. Vaccin comportant un colombopoxvirus Nakano vivant, dans lequel est insérée la totalité ou une partie d'une séquence de nucléotides codant une hémagglutinine neuraminidase dérivée du virus de la maladie de Newcastle, dans une région du génome non-essentielle pour la prolifération du colombopoxvirus Nakano.

7. Vaccin conforme à l'une des revendications 1 à 6, qui est capable de conférer à des volailles une immunité antivirale contre le poxvirus des volailles et contre le virus de la maladie de Newcastle.

8. Emploi d'un avipoxvirus recombinant, tel qu'il est défini dans l'une des revendications 1 à 6, dans la préparation d'un vaccin destiné à des volailles.

9. Emploi d'un avipoxvirus recombinant, tel qu'il est défini dans l'une des revendications 1 à 6, dans la préparation d'un vaccin destiné à des volailles, capable de conférer à des volailles une immunité antivirale contre le poxvirus des volailles et contre le virus de la maladie de Newcastle.
